# EUROPEAN PATENT APPLICATION

(11) **EP 4 368 150 A1**
(43) Date of publication of application: **15.05.2024**
(21) Application number: 22207038.5
(22) Date of filing: 11.11.2022
(51) Int. Cl.: A61F 2/50, A61F 2/68, A61F 2/70, A61F 2/76

(54) **THERMAL SENSING DEVICE AND SENSORY FEEDBACK SYSTEM AND METHOD USING SUCH THERMAL SENSING DEVICE**

(71) Applicant: ECOLE POLYTECHNIQUE FEDERALE DE LAUSANNE (EPFL), 1015 Lausanne (CH); Scuola Superiore di Studi Universitari e di Perfezionamento Sant'Anna, 56127 Pisa (IT)
(72) Inventor: Shokur, Solaiman, 1206 Genève (CH); Akouissi, Outman, 1004 Lausanne (CH); Iberite, Francesco, 56126 Pise (IT); Muheim, Jonathan, 1183 Bursins (FR); Clerc, André, 1005 Lausanne (CH); Micera, Silvestro, 1204 Genève (CH)
(74) Representative: Debay, Damien

(57) **Abstract**

The present invention concerns a thermal sensing device (1) and a sensory feedback system and method using such thermal sensing device, comprising at least one film (19) of electrically insulating polymer defining a global surface of the thermal sensing device (1) and including at least one sensing track (10) of a conducting material exhibiting a change in resistivity with temperature, said sensing track (10) being arranged with a connection (12) to at least one control module (2) receiving the signal of the thermal sensing device (1) as a measure of temperature, said thermal sensing device (1) being characterized in that said film (19) further comprises at least one heating track (11), powered by said control module (2) to dissipate electrical power into heat within the thermal sensing device (1) and maintain the sensing track (10) at a determined baseline temperature, preferably close to the baseline temperature of the human skin.

## Description

### Field of the invention

The present invention concerns the field of neurosciences and in particular of haptic perception. More precisely, the invention relates to the conscious thermal perception.

In particular, the present invention concerns a thermal sensing device and a sensory feedback system and method using such thermal sensing device.

### Background of the invention

In the field of thermal perception, several attempts have been made to provide devices and system allowing to provide an accurate detection of temperature variations, so as to mimic the natural thermal sensing ability of the skin. Such detection of temperature variations can be used in sensory feedback by applying the detected temperature to subjects. Such feedback can be used to restore the perception of temperature of subject who have lost this perception on a part of their body, for any reason, or to provide this perception of temperature detected by an object manipulated by subjects who want to feel the temperature variations to which this object is submitted. However, one major problem is the time constant of the detectors, such as some thermistors used in the prior art, which is too long for providing an accurate information. The known thermal detectors often react too slowly, which adds an unwanted additional delay to the perception of temperature which is already quite long compared to other sensory modalities. Furthermore, the form factor of known sensors implies a sensing spot which is too small and the temperature measured is highly sensitive to the quality of the contact between the sensor and the object of which the measure of temperature is desired. The three main types of temperature sensors used in the art are: thermocouples, thermistors and resistance temperature detectors (RTD). Thermocouples rely on the Seebeck effect to sense differences of temperature. This thermoelectric effect is responsible for the generation of an electric potential difference between the ends of two conductive materials when heat energy is applied between them. They are usually best suited to measure stable reference temperatures. Thermistors are typically made of materials whose resistivity exhibits a high sensitivity to temperature. Thus, they allow to detect fine thermal changes. Another problem in the field of thermal feedback is to apply the detected temperature to the subjects. Generally, the solutions of the prior art don't allow to apply temperatures lower than 19°C because of the heat of the skin onto which the temperature has to be applied. Another problem for providing a thermal sensory feedback is the recognition, by the subjects, of the temperatures detected and applied to their skin. This third problem is linked to the two previous problems because of the drawbacks of the prior art concerning the speed of the temperature detection and the deficient application of temperature, but is also linked to the training of the subjects who needs to associate the stimuli with temperature so as to offer an accurate thermal perception.

### Summary of the invention

One purpose of the present invention is to overcome at least some drawbacks of the prior art by proposing a thermal sensing device (1) which has increased performances allowing thermal sensory feedback to users or subjects.

This purpose is reached by a thermal sensing device configured to mimic the thermal sensing ability of the human skin, comprising at least one film of electrically insulating polymer defining a global surface of the thermal sensing device and including at least one sensing track of a conducting material exhibiting a change in resistivity with temperature, said sensing track being operatively connected to at least one control module receiving the signal of the thermal sensing device as a measure of temperature, said thermal sensing device being characterized in that said film further comprises at least one heating track, powered by said control module to dissipate electrical power into heat within the thermal sensing device and to maintain the sensing track at a determined baseline temperature, preferably close to the baseline temperature of the human skin.

According to another feature, the heating track and the sensing track are interlaced and/or stacked within said polymer film without contacting each other and are each arranged in serpentines comprising several loops across the global surface of the thermal sensing device, at least part of the loops of the heating tracks surrounding and/or following at least part of the loops of the sensing tracks, or inversely.

According to another feature, the number of loops is equal or superior to two, in any area of less than one hundred square millimeters within the surface of the thermal sensing device, preferably in any area of less than fifty square millimeters, ideally twenty-five square millimeters.

According to another feature, the mass of the heating track is equal or inferior to four times the mass of the heating track on the global surface of the thermal sensing device, preferably equal or inferior to two times.

According to another feature, the mass of the heating track is equal or inferior to four times the mass of the sensing track, ideally equal or superior to two times, in any area of less than one hundred square millimeters within the surface of the thermal sensing device, preferably on any area of less than fifty square millimeters, ideally twenty-five square millimeters.

According to another feature, at least some of the loops are open toward the periphery of the surface of the thermal sensing device and define open portions of the tracks, in which the thickness of the thermal sensing device is cut so as improve its ability to be bent without braking and/or disturbing the temperature measurement.

According to another feature, the heating track has a convex hull intersecting a convex hull of the sensing track by at least ten percent of the global surface of the thermal sensing device, preferably more than fifty percent, ideally close to ninety percent.

According to another feature, the heating track and the sensing track have different resistances of the same order of magnitude, ranging from a few hundreds to a few thousands of ohms, with preferably each close to one of the extremes of the range, the resistance of the heating track being the lower resistance.

According to another feature, the thermal sensing device further includes a thermally conductive sheet, preferably at or close to the side of the thermal sensing device which is configured to be in contact with the environment or objects.

Another purpose of the present invention is also to propose a sensory feedback system overcoming some drawbacks of the prior art by providing an improved thermal sensory feedback system.

This purpose is reached by a sensory feedback system for providing at least a thermal sensory feedback signal from at least one remote location to at least one functional spot of a subject, said functional spot being an area of the body of the subject which is consciously responsive to temperature variations, said system comprising at least one thermal sensing device having a resistance placed at said remote location and connected to a control module converting the resistance signal provided by the thermal sensing device into a measured temperature value and providing a thermal stimulator with a current enabling said thermal stimulator to reach said temperature value, the thermal stimulator comprising, on one side, at least one Peltier element configured to be in thermal contact with said at least one functional spot and, on the opposite side, a heatsink dissipating the temperature outside the thermal stimulator, the system being characterized in that said thermal sensing device is a thermal sensing device according to any embodiment in the present application, which is powered by said control module controlling the power delivered to the heating track so as to maintain the sensing track at a determined baseline temperature, said control module measuring the variations of temperature of the sensing track to set the current value delivered to said thermal stimulator thereby reaching a desired temperature determined by the control module as a function of the temperature measured by the thermal sensing device.

According to another feature, said heatsink extends the exchange surface of said at least one Peltier element and is equipped with at least one thermal insulation element (31,310) positioned, when in use, between the skin of the subject and the parts of the heatsink surrounding said at least one Peltier element.

According to another feature, said thermal stimulator includes a thermal sensor measuring the temperature of said at least one Peltier element and providing this measured temperature to the temperature controller for setting the power to be applied.

According to another feature, said thermal stimulator includes two Peltier elements connected in series and mounted on the same heatsink, a thermally conductive sheet being disposed on the sides of the two Peltier opposite the heatsink so as to cover both Peltier elements and contact the skin when in use, while bearing said thermal sensor which is disposed between the two Peltier elements.

According to another feature, the sensory feedback system further comprises :
- In addition to the thermal sensing device, at least one other type of sensor, such as a pressure sensor, a vibrotactile sensor, a piezo-resistive sensor, a microelectric mechanic system (MEMS), a gauge meter, a gyroscope, a magnetic sensor, an electric condenser microphone, a tension sensor, or any combination thereof, or wherein the capacity of several sensors is combined in at least one multisensor ;
- In addition to the thermal stimulator, at least one other type of stimulator, such as electroactive polymers, piezoelectric elements or other types of vibrating elements, for instance DC motors or membranes according to the same principle as being used in loudspeakers, e.g. vibrating motors, miniature loudspeakers or voice coils ;
- An additional or modified control module adapted to receive the signal generated by said other type of sensor when subjected to at least one stimuli and to transfer a signal to said other type of stimulator which has the ability to transduce said signal to said stimuli on a function-al spot.

Another purpose of the present invention is also to propose a sensory feedback method overcoming some drawbacks of the prior art by providing an improved thermal sensory feedback method. This purpose is reached by a method for conscious sensory feedback for a body extremity without sensation or a lacking body extremity of a subject by using a system according to any embodiment in the present application, wherein it comprises the following steps:
a) applying said at least one thermal sensing device to a body extremity without sensation or a body extremity prosthesis of a subject at one or more locations where conscious sensory feedback is desired,
b) optionally identifying, optionally before step a), one or more functional spot locations on the skin of the subject,
c) applying at least one thermal stimulator to said at least one functional spot,
d) optionally repeatedly subjecting said at least one thermal sensing device to at least one stimuli inducing temperature variation, during visual observation and/or hearing by the subject until the subject has learned to recognize said at least one stimuli and to correlate it to said at least one functional spot,
   wherein when said at least one thermal sensing device is subjected to at least one stimuli, the subject perceives said at least one stimuli on the body extremity prosthesis or the body extremity without sensation.

According to another feature, the method is implemented in a system comprising several thermal sensing devices and several thermal stimulators which are arranged in a spatial pattern on several body functional spots corresponding to a spatial pattern arrangement of the thermal sensing devices which has been determined by a mapping of the functional spots by repeatedly performing step b) during visual observation and/or hearing of the subject.

### Brief description of the drawings

Various technical features and advantages of the present invention will appear more clearly by reading the description of various examples of embodiments below, made in reference to the illustrative and non-limiting drawings, among which:
[Fig 1] Figure 1 shows a schematic view of the system according to various embodiments of the present invention ;
[Fig 2] Figures 2A, 2B, 2C, 2D, 2E and 2F shows schematic views (upper views in Figures 2A to 2E and perspective view in figure 2F) of thermal sensing devices according to various embodiments of the present invention ;
[Fig 3] Figure 3A shows on schematic side view of a thermal stimulator according to various embodiments of the present invention, Figure 3B shows a lower view of the arrangement of Peltier elements in a thermal stimulator of Figure 3A and Figure 3C show a cut view of this arrangement according to plane 3C-3C of Figure 3B.

### Detailed description of embodiments

The present invention concerns a thermal sensing device, a system and a method using such thermal sensing device. The present application also describes an improved thermal stimulator used in the system, which can be an invention independently from the other components of the system, since this thermal stimulator overcomes drawbacks of the prior art only concerning the thermal stimulation. Therefore, the present invention may thus concern also a system and method including any thermal sensing device, such as those of the prior art, and a thermal stimulator as described in some embodiments of the present application.

In a general manner, the thermal sensing device (1) aims at mimicking the thermal sensing ability of the human skin. This thermal sensing device (1) comprises at least one film (19) of electrically insulating material, preferably a polymer, for example a thermoset, defining a global surface of the thermal sensing device (1) and including at least one sensing track (10) of a conducting material exhibiting a change in resistivity with temperature, said sensing track (10) being operatively connected through a connection (12) to at least one control module (2) receiving the signal of the thermal sensing device (1) as a measure of temperature, said thermal sensing device (1) being characterized in that said film (19) further comprises at least one heating track (11), powered by said control module (2) to dissipate electrical power into heat within the thermal sensing device (1) and to maintain the sensing track (10) at a determined baseline temperature, preferably close to the baseline temperature of the human skin.

The term "track" is used in the present application to designate a conductive material arrange as a thin (flat) layer having a thickness of twenty to four-hundred nanometers, preferably one hundred nanometers, and following an extended pattern covering a large compared to its thickness, for example an area between fifty and three hundred square millimeters, preferably at least one hundred square millimeters, ideally two hundred square millimeters. In fact, the sensing "track" is used as an RTD. It may be made or may comprise a metallic material with a resistivity displaying a highly linear relationship with temperature. Even if different materials are typically used (gold, silver, platinum, copper, nickel), platinum is usually chosen for its non-oxidation properties, high biocompatibility and good mechanical resistance. The fact that the track is thin and covers a large surface provides the device with the advantageous ability to react very fast to variations of temperature. Indeed, it has been discovered by such arrangement that the drawbacks of the prior art are at least partially due to the cylindrical shape and/or small extent of the sensing devices. Furthermore, the fact that the sensing track is closely associated with a heating track allows the measured temperature variations to be realistic (with reference to normal/physiological perception) and allows a uniform distribution of heat across the global surface of the thermal sensing device (1). In preferred embodiments, the tracks have a width comprised between fifty and five hundred micrometers, preferably between two hundred and five hundred micrometers. In some embodiments, the heating track (11) has a width of approximately five hundred micrometers while the sensing track (10) has a width of approximately two hundred and fifty micrometers. Such difference by a factor two might be of less or more than two but not more than four, so that the mass of the heating track (11) allows a correct heating of the sensing track (10) which mass remains sufficient for measuring the target temperatures comprised between 0 and 50°C, preferably between 15 and 45°C.

The thermal sensing device (1) is called Active Thermal Sensor (ATS) in the present application because it includes an active heating of itself. The purpose of the heating track is to maintain the ATS at a determined baseline temperature, which means a constant temperature under constant (external) conditions, such as ambient temperature, airflow and absence of contact with any other object. This temperature is preferably approximately around 32°C (+ or - 4 °C) which is the most realistic temperature because it corresponds to the baseline temperature of the skin.

However, an important feature is that this temperature is at least different from the ambient temperature, such that the sensing device allows to distinguish the condition when no object is in contact with the device from the condition when an object at room temperature is in contact with the device. Thus, this constant temperature can be varied according to the ambient temperature and the control module can integrate an offset to take this variation into account.

It should be noted that a baseline temperature of the ATS (1) close to the main temperature of the skin has the further advantage of providing a realistic temperature and thus results in a better sensation of the users or subjects who touche it. Thereby, a prosthesis wearing or including the ATS (1) will be felt as more natural by people touching it. Indeed, it is known in the art that the temperature is more important than the softness for the perception of an artificial skin to be felt real.

The connection (12) between the ATS (1) and the control module (2) is preferably a standardized connector for facilitating the connection, for example in case of displacement or replacement of the ATS (1).

The global surface of the ATS (1) may be comprised between fifty and three hundred square millimeters but preferably represents an area of at least one hundred square millimeters, ideally two hundred square millimeters. Its shape can be square or rectangular (with square or preferably curved angles) or circular or elliptic or oblong. The preferred shape being the one closer to the shape of a phalanx or finger's tip. In the context of fingers or any other part of the body to be equipped with the ATS (1), the convexity or concavity of the surface receiving the ATS (1) may imposed some constraints on the conformability of the ATS (1). Therefore, the ATS (1) is preferably composed of materials allowing a flexible deformation and/or of materials allowing at least a plastic deformation within a certain range. For the heating and sensing tracks, these is achieved by the small thickness (twenty to four hundred nanometers, preferably one hundred nanometers) of the tracks which can thus be deformed. However, for the whole ATS (1), some embodiments propose improvements thank to the presence of peripheral cuts (112) on the ATS (1), as detailed below.

Any electrically insulating polymer that can be deposited as film (19), preferably thin (for example from 1 to 100 µm in thickness) and is compatible with common microfabrication techniques is suitable for the fabrication of the ATS (1). The film (19) must be electrically insulating and mechanically resistant to manipulation during installation and abrasion during the ATS (1) operation on the prosthesis. Ideally, the external polymeric layer (meaning the side of the ATS (1) that is going to be in contact with the external environment) should be thermally conductive to achieve a low time constant for the ATS (1). This can be achieved by engineering substrate thickness (1-5 µm in the case of thermosets such as polyimide or parylene) or by the inclusion of thermally conductive fillers (for larger thicknesses, e.g. PDMS or other polymers, preferably based on silicone).

Multiple polymeric layers could be integrated into the ATS (1) to improve the functionality of the device. For example, an elastomeric support layer could be integrated to ease manipulation and add a more biomimetic skin-like feeling. Additionally, metallic or thermally conductive layers (18) could be overlaid on the ATS (1) to engineer heat distribution and improve the overall sensing performance. The ATS (1) may include an adhesive layer for its removable attachment to any device, such as prostheses or robotic equipment. Alternatively, it may be included directly in the structure of the prosthesis or robotic equipment bearing it. It can for example be included in the silicone, latex or rubber skin of the prosthesis, preferably as close as possible to the outer surface. On the side opposite the outer side configured to be in contact with the external environment or objects (the side of the adhesive layer or of the prosthesis or robotic equipment), thermally insulating materials such foams, aerogels and other polymers (pure or filled), could be integrated to reduce undesired heat conduction towards the prosthesis, reducing power consumption and improving the sensing capabilities of the ATS (1). Such support materials improve the tear and scratch-resistance of the ATS (1).

In preferred embodiments, the heating track (11) and the sensing track (10) are interlaced and/or stacked within said polymer film (19) without contacting each other and are each arranged in serpentines comprising several loops (100, 110) across the global surface of the thermal sensing device (1), at least part of the loops (110) of the heating tracks (11) surrounding and/or following at least part of the loops (100) of the sensing tracks (10), or inversely.

The term "loops" in the present application designate the fact that the serpentine tracks are turning several times across the global surface of the sensing device, for a better homogeneity of the heat distribution and better accuracy of sensing the temperature. Preferably, these turns generally comprise a majority of U-turn (or open loops/hairpin turns), with square or curved angles, even if a few turns at 90° or other angles may be present for the wiring purpose of the device for its connection to the control module (2). The heating (11) and sensing (10) tracks have two extremities which preferably each comprise a pad (111) for receiving a wire enabling their connection to the control module (2).

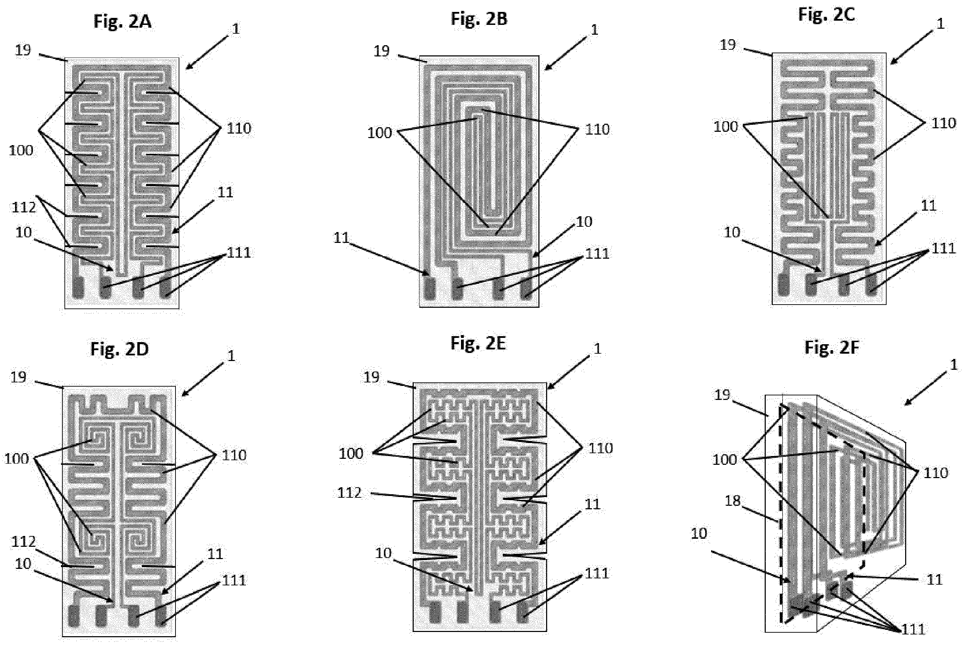

The figures 2A, 2B, 2C, 2D, 2E and 2F show examples of these preferred embodiments. In figures 2A, 2B, 2C, 2D and 2E, the heating (11) and sensing (10) tracks are both lying on the same plane and their loops surround each other. In figure 2F, the two tracks are in different stacked layers. In this example of Figure 2F, the patterns of the two tracks (10, 11) are the same, so that their respective loops follow exactly the same path. However, their patterns can be different as long as the proximity between them allow a uniform distribution of heat, so that the sensing track (10) is maintain at the correct temperature by the heating track (11). Furthermore, in the example of Figure 2F, the two tracks have the same width but they can be different as in the other figures for example. Conversely, the figures 2A, 2B, 2C, 2D and 2E show two tracks of different sizes but they may be the same, as explained below, in particular because of the concerns about the resistance and mass, as discussed above and below.

In some preferred embodiments, the distance between two neighboring portions of the heating track (11) is proportional, preferably equal, to the width of the heating track (11).

In some preferred embodiments, the distance between two neighboring portions of the sensing track (10) is proportional, preferably equal, to the width of the sensing track (10). In some preferred embodiments, the distance between two neighboring portions of the heating (11) and sensing (10) tracks is proportional, preferably equal, to the width of the heating (11) track and/or the sensing (10) track, preferably equal to the width of the sensing (10) track. In the case of stacked tracks like in Figure 2F for example, this distance is obtained by the layer interposed between the two tracks, which is more likely to ensure a good uniformity. This distance may be of the same order of the width of (at least one of) the tracks.

In some embodiments, the number of loops (100, 110) is equal or superior to two, in any area of less than one hundred square millimeters within the surface of the thermal sensing device (1), preferably in any area of less than fifty square millimeters, ideally twenty-five square millimeters. Indeed, it is preferred that both tracks make as many loops as possible within the global surface so that the heat is uniformly distributed by the heating track (11) and transferred to the sensing track (10).

In some embodiments, the mass of the heating (11) track is equal or inferior to four times the mass of the heating track (10) on the global surface of the thermal sensing device (1), preferably equal or inferior to two times. Indeed, it is advantageous that the heating track has a larger mass than the sensing track, for a better distribution of heat and a better influence on the sensing track (10).

In some preferred embodiments, the mass of the heating track (11) is equal or inferior to four times the mass of the sensing track (10), ideally equal or superior to two times, in any area of less than one hundred square millimeters within the surface of the thermal sensing device (1), preferably on any area of less than fifty square millimeters, ideally twenty-five square millimeters. Indeed, the fact that the distribution of the mass difference in the ATS is homogeneous helps to maintain a uniform heat and sensing accuracy of the sensing track (10).

In some embodiments, the heating track (11) has a convex hull intersecting a convex hull of the sensing track (10) by at least ten percent of the global surface of the thermal sensing device (1), preferably more than fifty percent, ideally close to ninety percent. For example, Fig. 2F illustrates two tracks that are stacked within the thickness of the film (19) which will then generally be thicker than in the embodiments having the two tracks in the same plane. In such case, the pattern of the two tracks can be completely identical, so that each loop of the heating track follows a loop of the sensing track. In such case, the projection of convex hull orthogonally to the planes of the tracks can intersect at one hundred percent.

In some embodiments, at least some of the loops (100, 110) are open toward the periphery of the surface of the thermal sensing device (1) and define open portions (111) of the tracks, in which the thick-ness of the thermal sensing device (1) is cut so as improve its ability to be bent without braking and/or disturbing the temperature measurement. The term "open portions" here designate portions of the ATS (1) in which no track is present and which a cut (112) can be performed in the thickness of the film (19), from the periphery toward the center of the ATS (1) without damaging the tracks, so as improve the ability of the ATS (1) to be bent and thus improve the conformability of the ATS (1) on a convex surface or even on a concave surface if the cuts (112) are more open at their outer end than at their inner end (for example as shown on figure 2E). These cuts (112) are shown on the lateral sides in the figures 2A, 2D and 2E but, in various embodiments, they can be performed on any side of the ATS (1).

In some embodiments, the heating track (11) and the sensing track have different resistances of the same order of magnitude, ranging from a few hundreds to a few thousands of ohms, with preferably each close to one of the extremes of the range, the resistance of the heating track (11) being the lower resistance. For example, with a thickness of one hundred nanometer and a width of two hundred and fifty micrometers, the sensing track (10) in Platinum will have a resistance between five hundred and one thousand and two hundred ohms, depending on the temperature, while the heating track (11) of the same thickness and a width of five hundred micrometers will have a resistance between two hundred and six hundred ohms, depending on the temperature. Such difference in resistance is linked to the difference in mass and advantageous by itself. Indeed, the resistance can be the same in the two tracks but it is preferred to widen the range of power that can be dissipated by the heating track, which implies to use lower resistances for limiting the power consumption, while it is also generally preferred to improve the sensitivity of the sensing track, which implies to use higher resistances. Therefore, a good compromise is found by having the resistances within the same range (and/or order of magnitude) but each one close to one extreme of the range. Furthermore, having such difference in resistance induces differences in mass when the two tracks are made in the same material, which provides the further advantage that the bigger heating track (11) more easily warms the smaller sensing track (10) up.

In some embodiments, the ATS (1) further includes a thermally conductive sheet (18), preferably at or close to the side of the thermal sensing device (1) which is configured to be in contact with the environment or objects. An example of such sheet (18) is shown in figure 2F which is more appropriate to show it because it is a perspective view, but such sheet (18) can be present in any pattern of the ATS (1). Such conductive sheet (18) is preferably covering substantially all the global surface and is advantageous because it helps to spread the temperature across ATS (1). In the embodiments having a sensing track and a heating track stacked one over the other, such conductive sheet (18) can be either on the side intended to be in contact with the environment of objects or be interposed between the sensing track and the heating track.

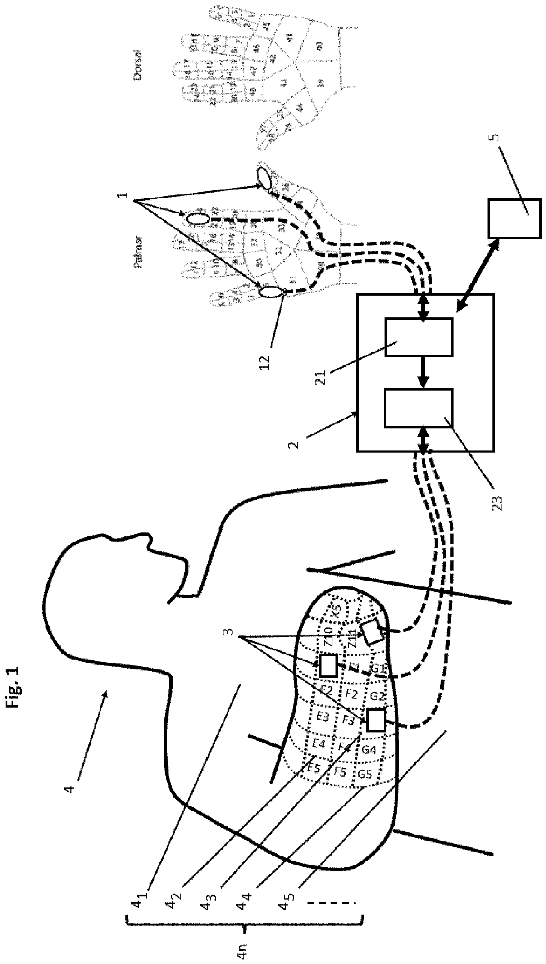

Some embodiments of the present invention concern a sensory feedback system for providing at least a thermal sensory feedback signal from at least one remote location to at least one functional spot (4n) of a subject (4), said functional spot (4n) being an area of the body of the subject (4) which is consciously responsive to temperature variations. An illustrative and non-limiting example of such system is shown in figure 1. Figure 1 show the example of an amputee (lacking the forearm in this example) for which the present invention is particularly advantageous but it can be applied to any subject (with impaired thermal sensation or not).

The functional spots can be of different locations (and/or nature) depending on the various possible applications of the present invention. One application is to restore the thermal sensation to a body part without sensation or a lacking body part or extremity, for example in amputees. One further application, in the case of multimodal sensors, is to improve the haptic perception of some sensors by providing additional thermal perception. Another application is to provide thermal sensations to any user (even unimpaired users, for example a surgeon) using a robotic equipment (for example a robotic hand) including the ATS (1) of the present invention, so that the user receives thermal information sensed by one or several ATS (1) thanks to the feedback provided by one or several thermal stimulator(s) placed on one or several functional spot(s) of the user, either on corresponding parts (like the fingers of the user, thanks to a glove, in the case of a robotic hand) or on other parts. Furthermore, in unimpaired subjects or in amputees, the functional spots can be at various locations, for example when some nerve derivation has been performed during the amputation (for example nerves of the arms derived to the chest) or when no functional spot is found on the remaining limb, the stimulation can be performed on the chest or the belly which proved to be very sensitive to temperature variations. The functional spots are thus very variable depending on the subjects and the applications of the present invention. They can be innate (mainly physiological sensing portions of the skin) or induced (mainly by surgery resulting in the cutting and/or derivation of nerve fibers, like in amputations) and/or acquired (by training the subject to the feedback stimulation received on any part of the skin). It will be noted that the accuracy of the ATS (1) of the present invention allowing discrimination of materials and recognition of wetness may also allow a machine learning of such discriminations and recognitions.

The system according to the present invention comprises at least one thermal sensing device (1) having a resistance placed at said remote location and connected to a control module (2) converting the resistance signal provided by the thermal sensing device (1) into a measured temperature value and providing a thermal stimulator (3) with a current enabling said thermal stimulator (3) to reach said temperature value, the thermal stimulator (3) comprising, on one side, at least one Peltier element (30) configured to be in thermal contact with said at least one functional spot (4n) and, on the opposite side, a heatsink (32) dissipating the temperature outside the thermal stimulator (3). More specifically, the system uses a thermal sensing device (1) according to any embodiments of the present invention, which is powered by said control module (2) controlling the power delivered to the heating track (11) so as to maintain the sensing track (10) at a determined baseline temperature, said control module (2) measuring the variations of temperature of the sensing track (10) to set the current value delivered to said thermal stimulator (3) thereby reaching a desired temperature determined by the control module (2) as a function of the temperature measured by the thermal sensing device (1).

In practice, the control module (2) can be one single device, such as a PID (Proportional Integral, Derivative) controller for controlling both the ATS (1) and the thermal stimulator (3). However, in some embodiments, for example as illustrated in a non-limiting manner in figure 1, the control module (2) includes, on one hand, at least one sensing controller (21) for controlling the power delivered to the ATS (1) for the heating track (11) and reading the temperature measured by the sensing track (10) and, on the other hand, at least one stimulation controller (23) controlling the Peltier element (30) for heating or cooling the functional spot. An illustrative and non-limiting example of such sensing controller (21) is the reference MKR WIFI 1010 of Arduino, while an illustrative and non-limiting example of such stimulation controller (23) is the reference TEC-1091 of Meerstetter Engineering, but many other types of controllers can be used for these two types of controller. Alternatively, a dedicated Printed Board Circuit (PCB) can be provided with the necessary functions of the control module (2). In addition, the control module (2) could also be split into two different modules, one for the measurements of temperature at the level of both the ATS (1) and the thermal stimulator (3) and one for delivering the necessary different currents to both the heating track (11) and the Peltier element (30). Also, the control module (2) can be split into four modules, one for each task of each (sensing and stimulating) device. In addition, the control module (2) can be connected to a computer device (5) which can set some of the values (for example of voltage or current) of the control module (2), but also to record the signals for future work. Optionally, the system may not comprise the thermal stimulator (3) for providing a feedback but just a computer device (5) to gather data acquired by the ATS and the control module during various experimental conditions submitted to the ATS (1). In some embodiments, said thermal stimulator (3) includes a thermal sensor (33) measuring the temperature of said at least one Peltier element and providing this measured temperature to the temperature controller (23) for setting the power to be applied. Alternatively, the stimulation controller (23) could be calibrated so that the current delivered to the Peltier element corresponds to a known target temperature, but such calibration is difficult and it is better safer to provide the system with a closing-loop sensor (33) ensuring that the correct temperature is applied. Furthermore, for safety reasons, the temperature range can thus be limited to values which will not be extreme, for example painful or in-convenient (generally between 15°C and 42 °C). Such thermal sensor (33) is preferably a Resistance Temperature Detector (RTD) suitable for the target temperatures. As a non-limiting example, the RTD manufactured by the company TE connectivity and reference as NB-PTCO-168 can be used.

It should be noted that the desired temperature to be reached by the Peltier element is determined as a function of the temperature by the ATS (1) and might be cold, cool, neutral, warm or hot. This desired or target temperature may thus present different ranges of temperatures which are in fact determined with reference to the baseline temperature of the ATS (1) (for the measured temperature) and to the baseline temperature of the thermal stimulator (3) (for the desired temperature). An offset between these temperatures is possible, thanks to the control module (2), but it is preferred that the temperature of the ATS (1) and of the thermal stimulator (3) are both at the baseline temperature of the skin. The measure of the temperature of the thermal sensor (33) of the thermal stimulator (33) may thus also be taken into account in the full closed loop of the system.

In some embodiments, said heatsink (32) extends the exchange surface of said at least one Peltier element and is equipped with at least one thermal insulation element (31,310) positioned, when in use, between the skin of the subject (4) and the parts of the heatsink (32) surrounding said at least one Peltier element (30).

It should be noted that the figures illustrate a passive heatsink but that other types of heatsink can be used. Furthermore, this heatsink shown is quite larger than the Peltier elements), but some smaller heatsink can be used, especially in the case of Phase Change Material heatsink for example or active heatsink. Such insulation preferably comprises a thermally conductive block (31) interposed between the Peltier element (30) and the heatsink (32), so that the latter is further away from the skin when in use. In addition or alternatively to this conductive block (31), such insulation can also include a thermally insulating layer (310) (preferably thinner than the Peltier element or than the Peltier and the conductive block), for example made of foam, disposed on the parts of the heatsink (32) surrounding said at least one Peltier element (on the side intended to be in contact with the skin). It will be noted that thanks to the insulation (31, 310) between the heatsink (32) and the skin allows to reach temperatures lower than 19°C, while the devices of previous designs didn't enable to the reach temperatures below 19°C.

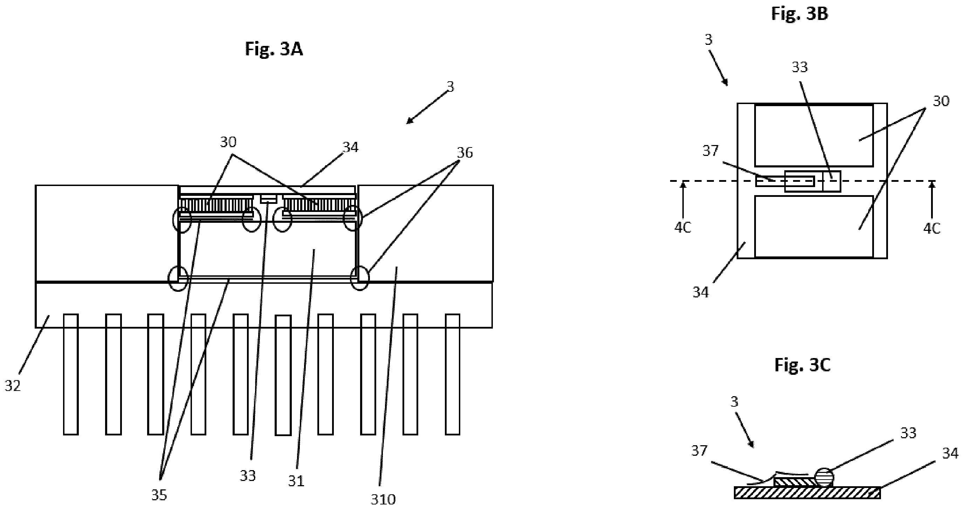

In some embodiments, said thermal stimulator (3) includes two Peltier elements (30) connected in series and mounted on the same heatsink (32), a thermally conductive sheet (34) being disposed on the sides of the two Peltier opposite the heatsink so as to cover both Peltier elements (30) and contact the skin when in use, while bearing said thermal sensor (33) which is disposed between the two Peltier element. An example of such thermal stimulator is shown in figures 3A, 3B and 3C. It will be understood that such arrangement extends to surface of contact with the skin and provides a better stimulation. Indeed, known Peltier elements are often a bit small for providing an accurate thermal stimulation and/or to bear a thermal sensor. These two problems are here solved at once by the two Peltier elements and the connecting conductive sheet bearing the RTD sensor. The size of the thermal stimulator will vary depending on the Peltier elements and the heatsink used. The surface of contact with the skin will be between forty to three hundred square millimeters. This contact surface may be square, rectangular, elliptic, circular or oblong and will preferably of approximately fifteen by fifteen square millimeters. This surface is preferably born by the conductive sheet, for example made of a one millimeter thick copper sheet.

The various elements of the thermal stimulator are thermally in contact with each other, so as to obtain an accurate spread and distribution of the temperature, preferably through the use of thermal paste (35). Furthermore, they are generally fixed to each other in series by a fixation (36), preferably by glue. However, the curing process of glue may tilt the thermal sensor (33), which is preferably flat. The thermal sensor (33) may thus have another fixation (37), such as cantilever pressing it against the Peltier element(s) directly or against the thermally conductive sheet (34), which is preferably made of copper. The use of solderable material may also be preferred for the conductive block (31) and the thermally conductive sheet (34) for soldering (36) the elements instead of gluing them (since some Peltier elements available on the market include some parts (for example in copper) allowing this soldering).

In some embodiments, the sensory feedback system further comprises :
- In addition to the thermal sensing device (1), at least one other type of sensor, such as a pressure sensor, a vibrotactile sensor, a piezo-resistive sensor, a microelectric mechanic system (MEMS), a gauge meter, a gyroscope, a magnetic sensor, an electric condenser microphone, a tension sensor, or any combination thereof, or wherein the capacity of several sensors is combined in at least one multi-sensor ;
- In addition to the thermal stimulator (3), at least one other type of stimulator, such as electroactive polymers, piezoelectric elements or other types of vibrating elements, for instance DC motors or membranes according to the same principle as being used in loudspeakers, e.g. vibrating motors, miniature loudspeakers or voice coils ;
- An additional or modified control module (2) adapted to receive the signal generated by said other type of sensor when subjected to at least one stimuli and to transfer a signal to said other type of stimulator which has the ability to transduce said signal to said stimuli on a functional spot.

Such embodiments have the advantage to provide a multimodal sensing experience which improves the sensing of various modalities. Indeed, many example of a synergistic effect of the temperature and the other haptic senses has been shown. As explained in Gallo et al. 2015: "Encoded and Crossmodal Thermal Stimulation through a Fingertip-Sized Haptic Display", Frontiers in robotics and AI, October 2015, volume 2, article 25, doi: 10.3389/frobt.2015.00025, including the references cited therein), further to both the influence of tactile cues on stiffness discrimination and of temperature on mechanoreceptor's sensitivity, the precision of stiffness perception as been shown to improve with increasing skin temperature. Several studies highlighted the effect of skin temperature on the sensitivity of most mechanoreceptors, as reflected by changes in their activation threshold. This effect of temperature on mechanoreceptors has been shown to have a direct influence on tactile acuity, the perception of roughness, or two-point discrimination thresholds. Interestingly, combined coding of mechanical and thermal stimulations could account for reported effects of skin temperature on object recognition by touch. In particular, the silver Thaler illusion, involving cold objects to feel heavier than warm objects of equal weight and dimensions, appears to derive from the effects of thermal gradients on slow adaptive mechanoreceptors of both type I or II (SAI and/or SAII). Furthermore, for the perception of complex features resulting from a multimodal haptic integration, the influence of skin temperature is more intricate. For example, a strong implication of temperature in the perception of wetness was observed with effects different to the ones observed for vibrotactile perception. In fact, warm and wet stimuli have been found to suppress the perception of skin wetness while on the contrary cool but dry objects were observed to evoke the perception of wetness.

In addition, it has been observed that the present invention allows the subjects to recognize different materials (for example plastic, glass and copper), thanks to the sensing ability to follow the transfer of heat in a short delay (in the order of one second or two, generally less than five) and the temperature of stabilization after contact with the test materials. Furthermore, the present invention, with its real-time (hundreds milliseconds at the level of the ATS (1)) feedback, also allows to recognize wetness which is characterized by quick drops in temperatures.

It can thus be understood that the present invention, when applied with multimodal sensors will have advantageously increased performances.

Some embodiments of the present invention concern a sensory feedback method for providing a conscious sensory feedback for a body extremity without sensation or a lacking body extremity of a subject by using a system according to any one of the embodiments described in the present application. Preferably, various embodiments of the method comprise the following steps:
a) applying said at least one thermal sensing device (1) to a body extremity without sensation or a body extremity prosthesis at one or more locations where conscious sensory feedback is desired,
b) optionally identifying, optionally before step a), one or more functional spot (4n) locations on the skin of the subject (4),
c) applying at least one thermal stimulator (3) to said at least one functional spot (4n),
d) optionally repeatedly subjecting said at least one thermal sensing device (1) to at least one stimuli inducing temperature variation, during visual observation and/or hearing by the subject until the subject has learned to recognize said at least one stimuli and to correlate it to said at least one functional spot (4n). According to these steps, when said at least one thermal sensing device (1) is subjected to at least one stimuli, the subject (4) perceives said at least one stimuli on the body extremity prosthesis or the body extremity without sensation.

Preferably, a training of the subject is performed, may the subject have an impairment in his thermal perception or not. Therefore, in some embodiments, the method comprises several thermal sensing devices (1) and several thermal stimulators (3) which are arranged in a spatial pattern on several body functional spots (4n) corresponding to a spatial pattern arrangement of the thermal sensing devices (1) which has been determined by a mapping of the functional spots (4n) by repeatedly performing step b) during visual observation and/or hearing of the subject.

The term "without sensation" used in the present application is intended to mean a body extremity which has reduced sensation, or no sensation and/or a part of a body extremity lacking or having reduced sensation. Body extremities may lack sensation, e.g. due to nerve injury or metabolic neuropathy. In such situations the body extremity is very vulnerable to injury since sensory feedback is lacking. The term "body extremity" used herein is intended to mean an arm or a leg or a part thereof, e.g. one or more fingers or toes or parts thereof, a whole hand or foot or a part thereof, the forearm or lower leg, such as the calf, or a part thereof and the upper arm or the thigh or a part thereof. The terms "body extremity prostheses" or "prostheses" used herein are intended to mean an artificial replacement of a body extremity as defined above and comprise conventional prostheses used as replacements for arms and legs and any parts thereof. In one embodiment said body extremity prostheses may have the whole system according to the present invention included.

However, the term "body extremity prostheses" also encompasses any type of object wearing the ATS (1) described in the present application, for example such as a pin or a pen. Indeed, anything on which such ATS (1) is placed can touch objects and detect their temperature and eventually recognize their material and/or wetness. Figure 1 shows, in an illustrative and non-limiting manner, an amputee having functional spots at its residual arm and on the chest and the belly, with thermal stimulators (3) connected to some functional spots identified according to a mapping within a temporary tattoo grid with correspondence to the naturally occurring spots in the hand (identified under supervision of the subject (4) prior to installation of the system). The figure 1 also shows some ATS (1) attached onto a prosthesis, in some areas corresponding to the locations of the identified functional spots. Such use of the present invention is particularly advantageous, but as explained above, the applications of the present invention are not limited to this example. The subject may for example have unimpaired arms and have a thermal sensory map of it hand similar to the one shown on the prosthesis of figure 1 which is therefor replicated onto a robotic hand. The corresponding thermal sensing devices (1) and thermal stimulators (3) will then be applied to, respectively, the robotic hand and the real hand according to this map. Alternatively, the ATS (1) may be installed on robotic hand controlled by a computer whjch receives the signals from the control module (2) so as to gather information, for example for a machine learning of the detection of temperature and/or the discrimination of materials or recognition of wetness.

The present application describes various technical features and advantages with reference to the figures and/or to various embodiments. Those skilled in the art will understand that the technical features of a given embodiment can indeed be combined with features of one or more other embodiment(s) unless the reverse is explicitly mentioned or these characteristics are incompatible or the combination does not work. In addition, the technical features described in a given embodiment can be isolated from the other features of this mode unless the reverse is explicitly mentioned, in particular because the functional considerations provided in the present application will provide a sufficient explanation so that the structural adaptations possibly necessary are within the reach of those skilled in the art. Therefore, the embodiments described in the present application should be considered by way of illustration and the invention should not be limited to the details given above.

### List of reference numbers

(1) thermal sensing device
(10) sensing track
(11) heating track
(100) sensing loop (of sensing track)
(110) loop (of heating track)
(111) pad
(12) connection
(18) thermally conductive sheet
(19) polymer film
(2) control module
(21) sensing controller
(23) stimulation controller
(3) thermal stimulator
(30) Peltier element
(31) conductive block
(310) insulation layer
(32) heatsink
(33) thermal sensor
(34) thermally conductive sheet
(35) thermal paste
(36) fixation
(37) cantilever
(4) subject
(4n) functional spots (n = 1 to a variable determined number)

## Claims

1. Thermal sensing device (1) configured to mimick the thermal sensing ability of the human skin, comprising at least one film (19) of electrically insulating material, preferably a polymer, for example a thermoset, defining a global surface of the thermal sensing device (1) and including at least one sensing track (10) of a conducting material exhibiting a change in resistivity with temperature, said sensing track (10) being operatively connected (12) to at least one control module (2) receiving the signal of the thermal sensing device (1) as a measure of temperature, said thermal sensing device (1) being **characterized in that** said film (19) further comprises at least one heating track (11), powered by said control module (2) to dissipate electrical power into heat with-in the thermal sensing device (1) and to maintain the sensing track (10) at a determined baseline temperature, preferably close to the baseline temperature of the human skin.

2. Thermal sensing device (1) according to claim 1, **characterized in that** the heating track (11) and the sensing track (10) are interlaced and/or stacked within said polymer film (19) without contacting each other and are each arranged in serpentines comprising several loops (100, 110) across the global sur-face of the thermal sensing device (1), at least part of the loops (110) of the heating tracks (11) sur-rounding and/or following at least part of the loops (100) of the sensing tracks (10), or inversely.

3. Thermal sensing device (1) according to claim 2, **characterized in that** the number of loops (100, 110) is equal or superior to two, in any area of less than one hundred square millimeters within the surface of the thermal sensing device (1), preferably in any area of less than fifty square millimeters, ideally twenty-five square millimeters.

4. Thermal sensing device (1) according to any one of claims 1 to 3, **characterized in that** the mass of the heating (11) track is equal or inferior to four times the mass of the heating track (10) on the global surface of the thermal sensing device (1), preferably equal or inferior to two times.

5. Thermal sensing device (1) according to any one of claims 1 to 4, **characterized in that** the mass of the heating track (11) is equal or inferior to four times the mass of the sensing track (10), ideally equal or superior to two times, in any area of less than one hundred square millimeters within the surface of the thermal sensing device (1), preferably on any area of less than fifty square millimeters, ideally twenty-five square millimeters.

6. Thermal sensing device (1) according to any one of claims 2 to 5, **characterized in that** at least some of the loops (100, 110) are open toward the periphery of the surface of the thermal sensing device (1) and define open portions (111) of the tracks, in which the thickness of the thermal sensing device (1) is cut so as improve its ability to be bent without braking and/or disturbing the temperature measurement.

7. Thermal sensing device (1) according to any one of claims 1 to 6, **characterized in that** the heating track (11) has a convex hull intersecting a convex hull of the sensing track (10) by at least ten percent of the global surface of the thermal sensing device (1), preferably more than fifty percent, ideally close to ninety percent.

8. Thermal sensing device (1) according to any one of claims 1 to 7, **characterized in that** the heating track (11) and the sensing track have different resistances of the same order of magnitude, ranging from a few hundreds to a few thousands of ohms, with preferably each close to one of the extremes of the range, the resistance of the heating track (11) being the lower resistance.

9. Thermal sensing device (1) according to any one of claims 1 to 8, **characterized in that** it further includes a thermally conductive sheet (18), preferably at or close to the side of the thermal sensing de-vice (1) which is configured to be in contact with the environment or objects.

10. Sensory feedback system for providing at least a thermal sensory feedback signal from at least one remote location to at least one functional spot (4n) of a subject (4), said functional spot (4n) being an area of the body of the subject (4) which is consciously responsive to temperature variations, said system comprising at least one thermal sensing device (1) having a resistance placed at said remote location and connected to a control module (2) converting the resistance signal provided by the thermal sensing device (1) into a measured temperature value and providing a thermal stimulator (3) with a current enabling said thermal stimulator (3) to reach said temperature value, the thermal stimulator (3) comprising, on one side, at least one Peltier element (30) configured to be in thermal contact with said at least one functional spot (4n) and, on the opposite side, a heatsink (32) dissipating the temperature outside the thermal stimulator (3), the system being **characterized in that** said thermal sensing device is a thermal sensing device (1) according to any one of claims 1 to 9, which is powered by said control module (2) controlling the power delivered to the heating track (11) so as to maintain the sensing track (10) at a determined baseline temperature, said control module (2) measuring the variations of temperature of the sensing track (10) to set the current value delivered to said thermal stimulator (3) thereby reaching a desired temperature determined by the control module (2) as a function of the temperature measured by the thermal sensing device (1).

11. Sensory feedback system according to claim 10, **characterized in that** said heatsink (32) extends the exchange surface of said at least one Peltier element and is equipped with at least one thermal insulation element (31, 310) between the skin of the subject (4) and the parts of the heatsink (32) surrounding said at least one Peltier element (30).

12. Sensory feedback system according to claim 10 or 11, **characterized in that** said thermal stimulator (3) includes a thermal sensor (33) measuring the temperature of said at least one Peltier element and providing this measured temperature to the temperature controller (23) for setting the power to be applied.

13. Sensory feedback system according to any one of claims 10 to 12, **characterized in that** said thermal stimulator (3) includes two Peltier elements (30) connected in series and mounted on the same heatsink (32), a thermally conductive sheet (34) being disposed on the sides of the two Peltier oppo-site the heatsink so as to cover both Peltier elements (30) and contact the skin when in use, while bearing said thermal sensor (33) which is disposed between the two Peltier elements.

14. Sensory feedback system according to any one of claims 10 to 13, **characterized in that** it further comprises :
- In addition to the thermal sensing device (1), at least one other type of sensor, such as a pressure sensor, a vibrotactile sensor, a piezo-resistive sensor, a microelectric mechanic system (MEMS), a gauge meter, a gyroscope, a magnetic sensor, an electric condenser microphone, a tension sensor, or any combination thereof, or wherein the capacity of several sensors is combined in at least one multi-sensor ;
- In addition to the thermal stimulator (3), at least one other type of stimulator, such as electroactive polymers, piezoelectric elements or other types of vibrating elements, for instance DC motors or membranes according to the same principle as being used in loudspeakers, e.g. vibrating motors, miniature loudspeakers or voice coils ;
- An additional or modified control module (2) adapted to receive the signal generated by said other type of sensor when subjected to at least one stimuli and to transfer a signal to said other type of stimulator which has the ability to transduce said signal to said stimuli on a functional spot.

15. A method for conscious sensory feedback for a body extremity without sensation or a lacking body extremity of a subject by using a system according to any one of claims 10 to 14, wherein it comprises the following steps:
a) applying said at least one thermal sensing device (1) to a body extremity without sensation or a body extremity prosthesis of a subject, at one or more locations where conscious sensory feedback is desired,
b) optionally identifying, optionally before step a), one or more functional spot (4n) locations on the skin of the subject (4),
c) applying at least one thermal stimulator (3) to said at least one functional spot (4n),
d) optionally repeatedly subjecting said at least one thermal sensing device (1) to at least one stimuli inducing temperature variation, during visual observation and/or hearing by the subject until the subject has learned to recognize said at least one stimuli and to correlate it to said at least one functional spot (4n),
wherein when said at least one thermal sensing device (1) is subjected to at least one stimuli, the subject (4) perceives said at least one stimuli on the body extremity prosthesis or the body extremity without sensation.

16. Method according to claim 15, wherein it comprises several thermal sensing devices (1) and several thermal stimulators (3) which are arranged in a spatial pattern on several body functional spots (4n) corresponding to a spatial pattern arrangement of the thermal sensing devices (1) which has been determined by a mapping of the functional spots (4n) by repeatedly performing step b) during visual observation and/or hearing of the subject.
